# EUROPEAN PATENT APPLICATION

(11) **EP 3 248 588 A1**
(43) Date of publication of application: **29.11.2017**
(21) Application number: 16740244.5
(22) Date of filing: 21.01.2016
(51) Int. Cl.: A61K 8/49, A61K 8/36, A61K 8/365, A61K 8/92, A61Q 1/04, A61Q 1/08, A61Q 1/10

(54) **MAKEUP COSMETIC**

(30) Priority: 21.01.2015 JP 2015009871
(71) Applicant: Shiseido Company Ltd., Chuo-ku Tokyo 104-0061 (JP)
(72) Inventor: OOHASHI, Shihoka, Yokohama-shi Kanagawa 224-8558 (JP); HIRUMA, Yukiko, Yokohama-shi Kanagawa 224-8558 (JP)
(74) Representative: Merkle, Gebhard
(86) International application number: PCT/JP2016/051678
(87) International publication number: WO 2016/117637

(57) **Abstract**

An object of the present invention is to provide a novel makeup cosmetic successfully producing a bright fluorescent color and providing transparency (clearness). The present invention provides a makeup cosmetic comprising: (A) at least one or two or more oil-soluble dye selected from the group consisting of Red No. 218, Red No. 223 and Orange No. 201, (B) a higher fatty acid metal salt, and (C) an oily component, wherein a content of a pigment is 0.5 mass% or less; and the oil-soluble dye is dissolved or dispersed in the oily component. The makeup cosmetic of the present invention can produce a bright fluorescent color as well as transparency, and is suitable as a lipstick, a blusher or an eyeshadow.

## Description

### Technical Field

The present invention relates to a makeup cosmetic capable of emitting fluorescence to provide a bright (brilliant) fluorescent color and transparent (clear) finish. More specifically, the present invention relates to a cosmetic coloring material containing a predetermined material(s), an oil-soluble dye and an oily component in combination and a makeup cosmetic containing the cosmetic coloring material.

### Background Art

Makeup cosmetics including lip cosmetics for coloring lips such as lipsticks and glosses, rouges (blushers) and eye shadows exert important cosmetic effects in significantly changing the physical appearance of the user. The color of a cosmetic is a key factor in choosing a cosmetic product. Recently, it has been desired to develop makeup cosmetics having a bright color and giving a transparent (clear) impression; for example, a lipstick displaying a highly bright fluorescent color has attracted attention.

Makeup cosmetics usually contain an oil base and a coloring material (coloring agent). The coloring material is responsible for producing a color when the cosmetic is applied to the lips and skin. The coloring materials to be blended in cosmetics include organic synthetic coloring agents (also called as tar colors) and inorganic pigments. The organic synthetic coloring agents are classified into dyes and organic pigments. The organic pigments are further classified into pigment coloring agents themselves and lake pigments, which are prepared by making water-soluble or sparingly-water-soluble dyes to be water insoluble (Non Patent Document 1).

Patent Document 1 discloses that if a fluorescent lame (lame), which is prepared by adding flaky powder having at least one resin layer stained with a fluorescent dye or a fluorescent pigment, is blended in a makeup cosmetic, the fluorescent color of the resultant cosmetic is improved in uniformity and stability compared to the case in which a fluorescent dye or a fluorescent pigment is directly blended in a cosmetic.

However, it is considered that the color in appearance is determined by a pigment (Non Patent Document 1). The pigment, which is usually blended at 1 mass% or more, determines the color in appearance and also produces covering power. The cosmetic disclosed in Patent Document 1, which is prepared by adding a fluorescent lame containing a dye or pigment producing a fluorescent color to the color in appearance previously determined by a pigment, is interpreted as a makeup cosmetic for changing appearance. A dye or pigment intrinsically producing a fluorescent color is used as a material for the lame.

Patent Document 2 focuses on an oil-soluble dye for producing color of a lipstick cosmetic; however, Patent Document 2 only discloses a technique for blending perfluoropolyether in combination with an oil-soluble dye in order to solve problems, i.e., chapped lips and color change in appearance caused by dyeing of an oil-soluble dye conventionally used, but for a fluorescent color produced by the oil-soluble dye.

Patent Document 3 discloses that a makeup cosmetic (including a lipstick) containing an oil-soluble dye, which does not produce a fluorescent color when it is dissolved or dispersed alone in an oily component, in combination with a polyamide powder having a predetermined molecular weight and a predetermined particle diameter, produces a fluorescent color. However, the cosmetic containing an oil-soluble dye and a polyamide powder has a high covering power and lacks in transparency.

In short, a makeup cosmetic successfully producing a bright fluorescent color and providing transparency (clearness) has not yet been obtained up to present.

### Relevant Art Documents

### Patent Documents

Patent Document 1: JP-A 2004-346025
Patent Document 2: JP-B 3409191
Patent Document 3: JP-B 5312738

### Non Patent Document

Non Patent Document 1: "New Cosmetology (second edition)", edited by Takeo Mitsui, Nanzando Co., Ltd., 2001

### Summary of Invention

### Technical Problem

An object of the present invention is to provide a novel makeup cosmetic successfully producing a bright fluorescent color and providing transparency (clearness).

### Solution to Problem

The present inventors conducted intensive studies with a view to solving the aforementioned problems and, as a result, found that a makeup cosmetic producing a bright fluorescent color and providing excellent transparency (clearness) can be obtained by blending a specific soap-base material and a specific oil-soluble dye(s) in combination. Based on the finding, the present invention was accomplished.

More specifically, the present invention provides a makeup cosmetic containing (A) at least one oil-soluble dye selected from the group consisting of Red No. 218, Red No. 223 and Orange No. 201, (B) a higher fatty acid metal salt, and (C) an oily component, wherein the makeup cosmetic may further comprise a pigment in an amount of 0.5 mass% or less; and wherein the oil-soluble dye is dissolved or dispersed in the oily component.

The present invention further provides a cosmetic coloring material to be blended in the makeup cosmetic. The cosmetic coloring material of the present invention contains the components (A), (B) and (C).

### Advantageous Effects of Invention

The makeup cosmetic according to the present invention can produce a bright fluorescent color based on a fluorescent dye intrinsically not producing a fluorescent color by blending a specific oil-soluble dye(s) and a specific soap-base material in combination. The makeup cosmetic according to the present invention can not only provide excellent transparency (clearness) but also produce a bright fluorescent color by suppressing the content of a pigment to a predetermined value or less.

### Description of Embodiments

The present invention will be more specifically described below.

### (1) Makeup cosmetic

The makeup cosmetic of the present invention contains (A) an oil-soluble dye, (B) a higher fatty acid metal salt, and (C) an oily component, as essential components.

### (A) Oil-soluble dye

The oil-soluble dye (component A) to be blended in the makeup cosmetic of the present invention is selected from oil-soluble dyes of the coloring materials called organic synthetic coloring agents in the field of cosmetic, especially an oil-soluble dye of a fluorescein derivative, in particular, at least one or two or more selected from Red No. 218 (tetrachlorotetrabromofluorescein), Red No. 223 (tetrabromofluorescein) and Orange No. 201 (dibromofluorescein).

The content of the oil-soluble dye (component A) in the makeup cosmetic is preferably 0.0001 to 5.0 mass%, and more preferably 0.001% to 3.0 mass% relative to the whole cosmetic.

### (B) Higher fatty acid metal salt

The higher fatty acid metal salt (component B) to be blended in the makeup cosmetic of the present invention is a metal salt of a higher fatty acid having 6 or more carbon atoms. In the field of cosmetic, a salt of a higher fatty acid with an alkali metal such as sodium or potassium is generally called as a soap; whereas a salt of a higher fatty acid with a divalent or trivalent metal (non-alkali metal) such as calcium, zinc, magnesium or aluminum is called as a metal soap. The higher fatty acid metal salt of the present invention includes a soap and a metal soap.

The higher fatty acid constituting a higher fatty acid metal salt is preferably a linear or branched saturated or unsaturated higher fatty acid having about 6 to 24 carbon atoms. Examples thereof may include lauric acid, myristic acid, palmitic acid, stearic acid, isostearic acid and ricinoleic acid.

Examples of the metal forming a salt with the higher fatty acid as mentioned above may include alkali metals such as sodium and potassium; and divalent or trivalent non-alkali metals such as calcium, magnesium, lithium, barium and zinc. In the present invention, it is preferable to use a salt of a higher fatty acid with a non-alkali metal (metal soap).

Examples of the soap to be used in the present invention include sodium stearate and potassium laurate. Examples of the metal soap include lithium stearate, magnesium stearate, calcium stearate, aluminum stearate, barium stearate, zinc stearate, calcium laurate, barium laurate, zinc laurate, calcium ricinoleate and barium ricinoleate, but are not limited to these.

As the higher fatty acid metal salt in the present invention, any one of the aforementioned higher fatty acid metal salts may be used. Alternatively, a powder of an extender pigment such as talc, mica or sericite of which a surface is covered with any one of the aforementioned higher fatty acid metal salt may be used.

Note that if the higher fatty acid metal salt is powder, the shape and particle size thereof are not particularly limited and a powder usually used in cosmetics can be employed.

In the makeup cosmetic of the present invention, the content of the higher fatty acid metal salt (component B) relative to the whole cosmetic is preferably 0.001 to 30 mass% and more preferably 0.01 to 10 mass%.

The oily component (component C) to be blended in the makeup cosmetic of the present invention can be selected from oily ingredients usually blended in makeup cosmetics. As the oily component which can be blended, the following examples may be mentioned; however, the oily component is not limited to these.

As a solid oily component, solid fats, waxes, hydrocarbons and higher alcohols may be mentioned. Specific examples thereof include solid fats such as Japanese wax, cocoa butter and hardened castor oil; waxes such as carnauba wax, beeswax, candelilla wax and jojoba wax; hydrocarbon waxes such as polyethylene wax, paraffin wax, ceresin and microcrystalline wax; higher alcohols such as behenyl alcohol, cetanol and batyl alcohol; and silicone wax.

Examples of a liquid oily component include liquid oils such as olive oil, avocado oil, camellia oil, macadamia nut oil, evening primrose oil, jojoba oil, rapeseed oil, egg yolk oil, sesame oil, castor oil, safflower oil, cottonseed oil, soybean oil, tea seed oil, rice bran oil and germ oil; hydrocarbon oils such as squalane and liquid paraffin; ester oils such as isocetyl isostearate, cetyl 2-ethylhexanoate, 2-heptylundecyl palmitate, diisobutyl adipate, 2-hexyldecyl sebacate, isopropyl myristate, 2-octyldodecyl oleate, diisostearyl malate, ethylene glycol di-2-ethylhexanoate, glyceride tri-2-heptylundecanoate, glyceryl diisostearate, glyceryl triisostearate, glyceryl trioctanoate, glyceryl triisopalmitate, trimethylolpropane tri-2-ethylhexanoate and pentaerythritol tetra-2-ethylhexanoate; linear silicone oils such as dimethyl polysiloxane and methylphenyl polysiloxane; cyclic silicone oils such as octamethylcyclotetrasiloxane and decamethylcyclopentasiloxane; triglycerine; and fluorine-modified oils.

In the makeup cosmetic of the present invention, in addition to the above essential components (A) to (C), other components (hereinafter referred to also as "optional components"), which are usually blended in makeup cosmetics, can be blended as long as they do not negate the effect of the invention.

Examples of the optional components include components constituting a coloring material and an oil base of a makeup cosmetic and other (optional) components.

Examples of the component constituting a coloring material include an organic synthetic coloring agent (tar color) selected from organic dyes (excluding the oil-soluble dye serving as component A) and organic pigments (including pigment dyestuff and lake); natural colorants; and inorganic pigments (color pigments such as red iron oxide, yellow iron oxide and ultramarine blue, and white pigments such as titanium dioxide and zinc oxide).

Note that, in the makeup cosmetic of the present invention, in order to produce excellent transparency (clearness), the content of a pigment (organic pigment and inorganic pigment) relative to the whole cosmetic is preferably 0.5 mass% or less and further preferably 0.4 mass% or less.

As the component constituting the oil base, an oil thickener or a gelling agent may be mentioned.

Examples of the oil thickener (gelling agent) may include dextrin fatty acid esters, glycerin fatty acid ester and organic modified clay minerals. Examples of the dextrin fatty acid ester include dextrin myristate, dextrin palmitate and dextrin (palmitate/2-ethylhexanoate). Examples of the glycerin fatty acid ester include glyceryl behenate, glyceryl octastearate and glyceryl eicosanoate.

Examples of the other (optional) components include a UV absorber, a surfactant, an antioxidant, a fragrance, a preservative, a polyhydric alcohol, a lower alcohol, an inorganic powder (including a silica powder, a extender pigment, pearlescent pigment and a functional pigment such as photochromic pigment), an organic powder, and various active components.

Note that, in the present invention, the extender pigment of which a surface is coated with a higher fatty acid metal salt serving as component B belongs to component B and is excluded from the extender pigment.

The content(s) of these optional components can be appropriately controlled depending upon e.g., form and function of the makeup cosmetic.

The makeup cosmetic of the present invention exerts a special effect that never exerted before, i.e., not only producing a highly bright fluorescent color but also providing excellent transparency (clearness). Because of this, the makeup cosmetic of the present invention is particularly suitable for point-makeup cosmetics including lip cosmetics such as a lipstick and gross, blusher and eye shadow; and especially for an oily makeup cosmetic. Note that, lip creams and makeup bases and foundations which are not primarily intended to produce a color are not included in the makeup cosmetics of the present invention.

The makeup cosmetic of the present invention can be produced by a conventional method usually used for makeup cosmetics. More specifically, the makeup cosmetic of the invention can be produced by adding the essential components (A) to (C) and an optional component(s), mixing/stirring by e.g., a homo mixer while heating, and pouring the mixture into a mold followed by cooling. Preferably, a cosmetic coloring material is produced in advance by stirring/mixing the essential components (A) to (C) while heating, and then subjected to the conventional process together with other components to produce a makeup cosmetic.

### (2) Cosmetic coloring material

The present invention also provides a cosmetic coloring material particularly suitable for blending in the aforementioned makeup cosmetics. The cosmetic coloring material of the present invention contains an oil-soluble dye ("component A"), a higher fatty acid metal salt ("component B") and an oily component ("component C").

In the cosmetic coloring material of the present invention, the content of the oil-soluble dye (component A) relative to the whole coloring material is preferably 0.0002 to 10 mass% and more preferably 0.0005 to 8 mass%.

In the cosmetic coloring material of the present invention, the content of the higher fatty acid metal salt (component B) relative to the whole coloring material is preferably 0.002 to 60 mass% and more preferably 0.005 to 50 mass%.

The oily component serving as a constituent of the cosmetic coloring material of the present invention may constitute a part or whole of the oily component (component C) to be blended in the makeup cosmetic of the present invention and is preferably selected from the aforementioned oils that can dissolve or disperse the oil-soluble dye (component A).

Examples of the oily component suitably used for the coloring material of the present invention may include, but are not limited to, paraffin, squalane, isoparaffin, dimethicone, methylphenylpolysiloxane, di(isostearyl/phytosteryl) dilinoleate dimer, phytosteryl/behenyl dilinoleate dimer, phytosteryl/octyldodecyl lauroyl glutamate, glyceryl tri2-ethylhexanoate, glyceryl triisostearate, glyceryl tri(hydrogenated rosin/isostearate), diisostearyl malate, pentaerythrityl tetraethylhexanoate, dipentaerythrityl hexahydroxystearate and polyglyceryl-2 triisostearate.

In the cosmetic coloring material of the present invention, the content of the oily component relative to the whole coloring material is preferably 30 to 99.9978 mass% and more preferably 42 to 99.9945 mass%.

If necessary, e.g., an oil thickener (gelling agent) as mentioned above may be contained.

The cosmetic coloring material of the present invention is preferably contained at 10 to 60 mass%, and preferably 20 to 50 mass% in the makeup cosmetic producing a fluorescent color to which the cosmetic coloring material is added.

The coloring material of the present invention can be produced by, for example, dissolving or dispersing an oil-soluble dye (component A) in an oily component (component C), adding a higher fatty acid metal salt (component B) to the mixture and stirring/mixing the mixture while heating. The heating temperature preferably falls within the range of 80°C to 140°C, more preferably 90°C to 130°C and further preferably 100°C to 120°C.

In the coloring material and makeup cosmetic containing the coloring material of the present invention, an oil-soluble dye (component A) is present in an oily component (component C) in the state of being directly dissolved or dispersed, and can be clearly distinguished from a coloring material and a cosmetic containing, e.g., an oil-soluble dye carried on a solid carrier (e.g., solid particle).

### Examples

Now, the present invention will be more specifically described by way of Examples; however, the present invention is not limited by these. Note that, unless otherwise specified, the content is expressed by mass% relative to the whole amount.

### (Examples and Comparative Examples)

Lipstick cosmetics having the compositions listed in the following Table 1 and Table 2 were prepared.

In each example, lipstick cosmetics were prepared in accordance with the following two processes.

(Process 1) The components to be blended (listed in Tables) were combined, heated to 80 to 140°C, stirred/mixed, defoamed, poured in lipstick containers and cooled to 5°C.

(Process 2) Of the components listed in Tables, paraffin, microcrystalline wax, methylphenylsilicone, dye (Red No. 218) and a higher fatty acid metal salt (or other powders) were used in their whole amounts. The dye was dissolved in the oily component and the higher fatty acid metal salt or other powders were added, stirred/mixed while heating to 100 to 120°C to prepare a cosmetic coloring material. Thereafter, the resultant cosmetic coloring material and the remaining components were mixed/stirred and defoamed while heating to 80 to 140°C, and cooled to 5°C.

The lipstick cosmetics and coloring materials of individual examples were evaluated by 10 experts (panelists) in accordance with the following criteria.

### (1) Fluorescent color

Remarkable fluorescent color is observed: 4
Fluorescent color is observed: 3
Color is produced but not a fluorescent color: 2
Color is not observed: 1

### (2) Transparency (clearness)

Remarkably transparent (clear): 4
Transparent (clear): 3
Slightly transparent (clear): 2
Not transparent (clear) (covering power is strong): 1

Evaluation results according to the above criteria were rated as follows and the rating results of individual examples are shown together in Table 1 and Table 2.
A: Total score is 35 to 40
   (as to the fluorescent color, a case obtaining a total score of 35 to 37 was rated as A⁻; and a case obtaining a total score of 38 to 40 was rated as A⁺)
B: Total score is 25 to 34
C: Total score is 15 to 24
D: Total score is 10 to 14

**[Table 1]**

| | Component name | Example | | | | | |
|---|---|---|---|---|---|---|---|
| | | 1 | 2 | 3 | 4 | 5 | 6 |
| Solid oily component | Paraffin | 4 | 4 | 4 | 4 | 4 | 4 |
| | Microcrystalline wax | 2 | 2 | 2 | 2 | 2 | 2 |
| | Candelilla wax | 1 | 1 | 1 | 1 | 1 | 1 |
| Liquid oily component | Distearyl malate | 20 | 20 | 20 | 20 | 20 | 20 |
| | Methylphenylsilicone | 41.9 | 41.9 | 41.9 | 41.9 | 41.9 | 41.8 |
| | Hydrogenated polyisobutene | 10 | 10 | 10 | 10 | 10 | 10 |
| | Di(isostearyl/phytosteryl) dilinoleate dimer | 10 | 10 | 10 | 10 | 10 | 10 |
| | Polyglyceryl-2 triisostearate | 10 | 10 | 10 | 10 | 10 | 10 |
| Dye | Red No. 218 | 0.1 | 0.1 | 0.1 | 0.1 | 0.1 | 0.1 |
| Pigment | Red No. 202 | - | - | - | - | - | 0.1 |
| Higher fatty acid metal salt | Calcium stearate | 1 | - | - | - | - | 1 |
| | Aluminum stearate | - | 1 | - | - | - | - |
| | Magnesium stearate | - | - | 1 | - | - | - |
| | Zinc myristate | - | - | - | 1 | - | - |
| | Talc coated with calcium stearate | - | - | - | - | 1 | - |
| Other powders | Nylon | - | - | - | - | - | - |
| | Stearic acid | - | - | - | - | - | - |
| | Myristic acid | - | - | - | - | - | - |
| | Stearyl alcohol | - | - | - | - | - | - |
| Total | | 100 | 100 | 100 | 100 | 100 | 100 |
| Fluorescent color | Process 1 | A⁻ | A⁻ | A⁻ | A⁻ | A⁻ | A⁻ |
| | Process 2 | A⁺ | A⁺ | A⁺ | A⁺ | A⁺ | A⁺ |
| Transparency (clearness) | | A | A | A | A | A | A |

**[Table 2]**

| | Component name | Comparative Example | | | | |
|---|---|---|---|---|---|---|
| | | 1 | 2 | 3 | 4 | 5 |
| Solid oily component | Paraffin | 4 | 4 | 4 | 4 | 4 |
| | Microcrystalline wax | 2 | 2 | 2 | 2 | 2 |
| | Candelilla wax | 1 | 1 | 1 | 1 | 1 |
| Liquid oily component | Distearyl malate | 20 | 20 | 20 | 20 | 20 |
| | Methylphenylsilicone | 41.9 | 41.9 | 41.9 | 41.9 | 41.1 |
| | Hydrogenated polyisobutene | 10 | 10 | 10 | 10 | 10 |
| | Di(isostearyl/phytosteryl) dilinoleate dimer | 10 | 10 | 10 | 10 | 10 |
| | Polyglyceryl-2 triisostearate | 10 | 10 | 10 | 10 | 10 |
| Dye | Red No. 218 | 0.1 | 0.1 | 0.1 | 0.1 | 0.1 |
| Pigment | Red No. 202 | - | - | - | - | 0.8 |
| Higher fatty acid metal salt | Calcium stearate | - | - | - | - | 1 |
| | Aluminum stearate | - | - | - | - | - |
| | Magnesium stearate | - | - | - | - | - |
| | Zinc myristate | - | - | - | - | - |
| | Talc coated with calcium stearate | - | - | - | - | - |
| Other powders | Nylon | 1 | - | - | - | - |
| | Stearic acid | - | 1 | - | - | - |
| | Myristic acid | - | - | 1 | - | - |
| | Stearyl alcohol | - | - | - | 1 | - |
| Total | | 100 | 100 | 100 | 100 | 100 |
| Fluorescent color | Process 1 | B | C | C | C | C |
| | Process 2 | B | C | C | C | C |
| Transparency (clearness) | | C | C | C | C | C |

The cosmetic coloring materials of Examples 1 to 6 containing a higher fatty acid metal salt and prepared in accordance with Process 2 produced bright fluorescent colors and had clearness (evaluation of fluorescent color: A⁺); however, the coloring material of Comparative Example 1 containing a nylon powder (nylon SP-500, manufactured by Toray Industries Inc., molecular weight: 10,000 to 100,000, particle size: 10 µm or less, spherical powder) described in Patent Document 3 produced pink fluorescent color but poor, and hiding power was too strong to obtain transparency (clearness) (evaluation of fluorescent color: B). The cosmetic coloring materials of Comparative Examples 2 to 4 containing a higher fatty acid, which is solid at normal temperature, or a higher alcohol in place of a higher fatty acid metal salt failed to even produce a color (evaluation of fluorescent color: C).

As is apparent from the results shown in Table 1, in the makeup cosmetics of Examples 1 to 6 containing a specific oil-soluble dye (component A) and a higher fatty acid metal salt (component B), a bright fluorescent color was observed regardless of the process and transparency (clearness) of the cosmetics was excellent. Note that, the fluorescent colors in the cases where makeup cosmetics were produced in accordance with Process 2 were brighter.

In contrast, in the makeup cosmetics of Comparative Examples 1 to 4 containing another powder such as a nylon powder in place of a higher fatty acid salt and produced in accordance with either of Process 1 and Process 2, the fluorescent color produced was poor or no fluorescent color was produced, and no transparency (clearness) was obtained. In the makeup cosmetics of Comparative Example 5 containing more than 0.5 mass% of an organic pigment (Red No. 202), neither transparency (clearness) nor fluorescent color were obtained.

Other formulations (Examples 7 to 11) of the makeup cosmetic (lipstick) of the present invention will be described below. In any of Examples, a highly bright fluorescent color and transparency (clearness) were obtained. The evaluation results in Table are based on the aforementioned criteria.

Note that, the fluorescent colors of the makeup cosmetics produced in accordance with Process 1 (conventional method) were rated as "A⁻"; whereas the fluorescent colors of the makeup cosmetics produced in accordance with Process 2 were rated as "A⁺".

**[Table 3]**

| | Component name | Comparative Example | | | | |
|---|---|---|---|---|---|---|
| | | 1 | 2 | 3 | 4 | 5 |
| Solid oily component | Paraffin | 4 | 4 | 4 | 4 | 4 |
| | Microcrystalline wax | 2 | 2 | 2 | 2 | 2 |
| | Candelilla wax | 1 | 1 | 1 | 1 | 1 |
| Liquid oily component | Distearyl malate | 20 | 20 | 20 | 20 | 20 |
| | Methylphenylsilicone | 41.9 | 41.9 | 41.9 | 41.9 | 41.1 |
| | Hydrogenated polyisobutene | 10 | 10 | 10 | 10 | 10 |
| | Di(isostearyl/phytosteryl) dilinoleate dimer | 10 | 10 | 10 | 10 | 10 |
| | Polyglyceryl-2 triisostearate | 10 | 10 | 10 | 10 | 10 |
| Dye | Red No. 218 | 0.1 | 0.1 | 0.1 | 0.1 | 0.1 |
| Pigment | Red No. 202 | - | - | - | - | 0.8 |
| Higher fatty acid metal salt | Calcium stearate | - | - | - | - | 1 |
| | Aluminum stearate | - | - | - | - | - |
| | Magnesium stearate | - | - | - | - | - |
| | Zinc myristate | - | - | - | - | - |
| | Talc coated with calcium stearate | - | - | - | - | - |
| Other powders | Nylon | 1 | - | - | - | - |
| | Stearic acid | - | 1 | - | - | - |
| | Myristic acid | - | - | 1 | - | - |
| | Stearyl alcohol | - | - | - | 1 | - |
| Total | | 100 | 100 | 100 | 100 | 100 |
| Fluorescent color | Process 1 | B | C | C | C | C |
| | Process 2 | B | C | C | C | C |
| Transparency (clearness) | | C | C | C | C | C |

Other formulations of the makeup cosmetics according to the present invention will be described below. In any of the makeup cosmetics, bright fluorescent color and clearness were obtained.

**Formulation 1: Lip gloss**

| Component | Content (mass%) |
|---|---|
| 1. Liquid paraffin | 10.0 |
| 2. Polyisobutene | 10.0 |
| 3. Diisostearyl malate | 10.0 |
| 4. Dextrin palmitate | 10.0 |
| 5. Calcium stearate | 0.5 |
| 6. Orange No. 201 | 0.2 |
| 7. Iron oxide | 0.1 |
| 8. Red No. 202 | 0.1 |
| 9. Titanium mica | 2.0 |
| 10. Tocopherol | proper amount |
| 11. Fragrance | proper amount |
| 12. Diphenylsiloxy phenyl trimethicone | balance |

### (Production method)

Components 1 to 5 and 8 (each in the whole amount) were combined and stirred/mixed while heating to 100 to 120°C to prepare a cosmetic coloring material. Thereafter, the resultant cosmetic coloring material and the remaining components (6, 7 and 9 to 12) were combined, mixed/stirred and defoamed while heating to 80 to 140°C, and cooled.

**Formulation 2: Eye shadow**

| Component | Content (mass%) |
|---|---|
| 1. Talc | balance |
| 2. Mica | 20.0 |
| 3. Boron nitride | 10.0 |
| 4. Synthetic mica | 10.0 |
| 5. Mica coated with titanium oxide | 5.0 |
| 6. Iron oxide | 2.0 |
| 7. Squalane | 2.0 |
| 8. Diethyl polysiloxane | 2.0 |
| 9. Sorbitan monooleate | 0.5 |
| 10. Red No. 218 | 0.01 |
| 11. Aluminum stearate | 0.2 |
| 12. Preservative | proper amount |
| 13. Fragrance | proper amount |

### (Production method)

Components 1 to 6, 12 and 13 were ground and mixed. To this, a cosmetic coloring material prepared by mixing Components 7 to 11 while heating at 100 to 120°C was added and molded in a pan to obtain an eye shadow.

**Formulation 3: Blusher**

| Component | Content (mass%) |
|---|---|
| 1. Talc | balance |
| 2. Polymethyl methacrylate | 1.0 |
| 3. Silica | 2.0 |
| 4. Boron nitride | 3.0 |
| 5. Mica | 10.0 |
| 6. Isoparaffin | 3.0 |
| 7. Glyceryl tri-2-ethylhexanoate | 1.0 |
| 8. Sorbitan monooleate | 1.0 |
| 9. Iron oxide | 3.0 |
| 10. Red No. 223 | 0.1 |
| 11. Red No. 218 | 0.02 |
| 12. Magnesium stearate | 1 |
| 13. Preservative | proper amount |
| 14. Fragrance | proper amount |

### (Production method)

Components 1 to 5, 9, 13 and 14 were ground and mixed. To this, a cosmetic coloring material prepared by mixing components 6 to 8 and 10 to 12 while heating at 100 to 120°C, was added and molded in a pan to obtain a blusher.

## Claims

1. A makeup cosmetic comprising
(A) at least one oil-soluble dye selected from the group consisting of Red No. 218, Red No. 223 and Orange No. 201,
(B) a higher fatty acid metal salt, and
(C) an oily component,
wherein said makeup cosmetic may further comprise a pigment in an amount of 0.5 mass% or less; and wherein said oil-soluble dye is dissolved or dispersed in said oily component.

2. The makeup cosmetic according to Claim 1, wherein said higher fatty acid metal salt (B) is at least one metal soap selected from the group consisting of lithium stearate, magnesium stearate, calcium stearate, aluminum stearate, barium stearate, zinc stearate, calcium laurate, barium laurate, zinc laurate, calcium ricinoleate and barium ricinoleate.

3. The makeup cosmetic according to Claim 1 or 2, wherein the content of said oil-soluble dye (A) relative to a whole cosmetic is 0.0001 to 5 mass% and the content of said higher fatty acid metal salt (B) relative to the whole cosmetic is 0.001 to 30 mass%.

4. The makeup cosmetic according to any one of Claims 1 to 3, wherein: said makeup cosmetic is a lip cosmetic.

5. A coloring material for a makeup cosmetic comprising
(A) at least one oil-soluble dye selected from the group consisting of Red No. 218, Red No. 223 and Orange No. 201 at 0.0002 to 10 mass%,
(B) a higher fatty acid metal salt at 0.002 to 60 mass% and
(C) an oily component at 30 to 99.9978 mass%
wherein said oil-soluble dye is dissolved or dispersed in said oily component.

6. A makeup cosmetic containing the coloring material according to Claim 5.
